# EUROPEAN PATENT SPECIFICATION

(11) **EP 4 019 971 B1**
(45) Date of publication and mention of the grant of the patent: **08.11.2023**
(21) Application number: 20217463.7
(22) Date of filing: 28.12.2020
(51) Int. Cl.: G01N 33/543

(54) **LABEL-FREE ELECTROCHEMICAL BIOSENSOR FOR DETECTION OF BODYFLUID BASED BIOMARKERS**
MARKIERUNGSFREIER ELEKTROCHEMISCHER BIOSENSOR ZUM NACHWEIS VON BIOMARKERN AUF BASIS VON KÖRPERFLÜSSIGKEIT
BIOCAPTEUR ÉLECTROCHIMIQUE SANS ÉTIQUETTE POUR LA DÉTECTION DE BIOMARQUEURS À BASE DE FLUIDE CORPOREL

(43) Date of publication of application: 29.06.2022
(73) Proprietor: University of Plymouth, Plymouth PL4 8AA (GB)
(72) Inventor: Suhail, Ahmed, Plymouth, PL4 8AA (GB); Pan, Genhua, Plymouth, PL4 8AA (GB); Sethi, Jagriti, Plymouth, PL4 8AA (GB); Safarzadeh, Mina, Plymouth, PL4 8AA (GB)
(74) Representative: De Tullio, Michele Elio

(56) References cited:
- WO-A1-2019/023567
- WO-A1-2019/186354
- LINFEI LAI ET AL: "One-step synthesis of NH-graphene fromgraphene-oxide reduction and its improved electrochemical properties", CARBON, ELSEVIER OXFORD, GB, vol. 49, no. 10, 13 March 2011 (2011-03-13), pages 3250-3257, XP028218082, ISSN: 0008-6223, DOI: 10.1016/J.CARBON.2011.03.051 [retrieved on 2011-04-05]
- BARAKET MIRA ET AL: "Aminated graphene for DNA attachment produced via plasma functionalization", APPLIED PHYSICS LETTERS, A I P PUBLISHING LLC, US, vol. 100, no. 23, 4 June 2012 (2012-06-04) , pages 233123-233123, XP012156487, ISSN: 0003-6951, DOI: 10.1063/1.4711771 [retrieved on 2012-06-08]
- SETHI JAGRITI ET AL: "NH2 linker for femtomolar label-free detection with reduced graphene oxide screen-printed electrodes", CARBON, vol. 179, 26 April 2021 (2021-04-26), pages 514-522, XP055804866, GB ISSN: 0008-6223, DOI: 10.1016/j.carbon.2021.04.074

## Description

### TECHNICAL FIELD

The present invention relates to label-free biosensors for detection of body-fluid based biomarkers for disease diagnosis and a method to produce the same. In particular, the present invention relates to a new label-free biosensor comprising a novel amine (NH₂) linker, attached on a reduced graphene oxide (rGO) screen-printed electrodes (SPEs) and configured for the effective immobilisation of bioreceptors and for improving the performance of biosensors. A novel functionalization technique to attach the linker on the graphene-based sensor surfaces is presented.

The label-free detection of biomarkers employing the new linker is demonstrated using screen-printed rGO electrodes for the detection of Aβ₁₋₄₂ proteomic biomarkers and DNA methylation. The technique also applies to printed graphene electrodes.

### BACKGROUND ART

Biosensors are biochemical transducer comprising a biological sensitive element, called bio-receptors, capable of interacting with biological elements, and a transduction system aimed to convert this biochemical response into an electrical signal.

Critical aspect for the sensitivity of the biosensors is the ability of attaching the bioreceptors on the sensor surface, which ability can be achieved using "ad hoc" chemistry. Biosensors based on graphene and reduced graphene (rGO) platform have attracted enormous attention in recent years for their potential applications in detection of disease biomarkers from body fluids for non-invasive and cost-effective disease diagnosis. A variety of graphene-based biosensors that rely on different linker chemistries have been developed. However, one of the main challenges in making this technology clinically applicable is the need to improve sensing performance, in terms of sensitivity and reliability for the detection of biomarkers.

The state of the art in the field can be categorised in the following two main groups: label-free detection and labelled detection. Within the first category we find graphene-based label-free biosensors as described in the following papers:
1) Kawata, T., et al., Improved sensitivity of a graphene FET biosensor using porphyrin linkers. Japanese Journal of Applied Physics, 2018. 57(6): p. 065103. In this study, tetrakis (4-carboxyphenyl) porphyrin (TCPP) was used as a linker for surface modification of a Graphene FET (GFET). TCPP modification resulted in a higher density of receptor immunoglobulin E (IgE) aptamer molecules on the GFET. The detection limit of the target IgE was enhanced from 13 nM reached with the conventional linker to 2.2 nM.
2) Islam, K., et al., Development of a label-free immunosensor for clusterin detection as an alzheimer's biomarker. Sensors, 2018. 18(1): p. 308. In this paper the electrochemical detection of Clusterin (CLU), described as a potential Alzheimer's disease biomarker in blood plasma, is presented. A screen-printed carbon electrode (SPCE), modified with 1-pyrenebutyric acid N-hydroxysuccinimide ester (Pyr-NHS) and decorated with specific anti-CLU antibody fragments, is used as electrochemical biosensor. Cyclic voltammetric and square wave voltammetric studies showed the limit of detection down to 1 pg/mL.
3) Islam, K., A. Suhail, and G. Pan, A label-free and ultrasensitive Immunosensor for detection of human chorionic gonadotrophin based on graphene FETs. Biosensors, 2017. 7(3): p. 27. A label-free immunosensor based on graphene field effect transistors (GFETs) is presented, aimed for the detection of Human Chorionic Gonadotrophin (hCG), as an indicator of pregnancy and related disorders, such as actopic pregnancy, choriocarcinoma and orchic teratoma. A noncovalent bond between monolayer graphene and antibody was facilitated by applying a linker molecule known as 1-pyrenebutyric acid-N-hydroxysuccinimide ester (Pyr-NHS) via n-n bonding. The hCG concentration gradient showed a detection limit of ~1 pg/mL.
4) Chen, T.-Y., et al., Label-free detection of DNA hybridization using transistors based on CVD grown graphene. Biosensors and Bioelectronics, 2013. 41: p. 103-109. GFETs are used for label-free electrical detection of DNA hybridization. The gate materials, buffer concentration and surface condition of graphene have been optimized to achieve the DNA detection sensitivity as low as 1 pM (10⁻¹² M) .

In addition to the previous non-patent literature, the following patents and patent applications have been found describing graphene-based label-free biosensors.

For example, the US patent 10,465,244 B2 provides for a new electrochemical biosensor that uses DNA-redox electrostatic interaction and its subsequent non-specific adsorption on graphene screen-printed electrode or biochip. The invention provides new biomarkers to detect chicken and pork species with detection limit of 1 pg/µL and 100 pg/µL respectively.

For example, the US patent application US 2019187090 describes a biosensor having at least one working electrode covered with reduced graphene (rGO), wherein an antibody fragment Fab or another capture molecule is covalently bound to the reduced graphene via a spacer. The spacer preferably has terminal amino groups, one of these groups is used for attachment to the rGO, the other serves for covalent binding to the Fab. The object is to provide a biosensor with which an analyte molecule can be detected with particularly high sensitivity (the limit of detection is not reported) within an extremely short detection time.

In all these graphene-based label-free biosensors described in the foregoing, the maximum label-free detection sensitivity or Limit of Detection (LOD) achieved is around 1 pM.

Belonging to the second category, there are, for example, the labelled biosensing techniques described in the following papers:
1) Gagni, P., et al., Development of a high-sensitivity immunoassay for amyloid-beta 1-42 using a silicon microarray platform. Biosensors and Bioelectronics, 2013. 47: p. 490-495;
2) De la Escosura-Muñiz, A., et al., Alzheimer's disease biomarkers detection in human samples by efficient capturing through porous magnetic microspheres and labelling with electrocatalytic gold nanoparticles. Biosensors and Bioelectronics, 2015. 67: p. 162-169;
3) Wu, C.-C., et al., Electrochemical impedance spectroscopy analysis of A-beta (1-42) peptide using a nanostructured biochip. Electrochimica Acta, 2014. 134: p. 249-257.

These techniques achieve higher sensitivity (lower LOD) . However, they may involve more complex preparation process steps, being incompatible with mass-production or requiring complicated instrumentation.

For all the aforementioned reasons, there is a need of graphene-based label-free biosensors with improved sensing performance, both as sensitivity and reliability, for the detection of biomarkers.

### SUMMARY OF THE INVENTION

The aim of the present invention is consequently to provide an improved label-free graphene-based electrochemical biosensor.

In particular, the aim of the present invention is to provide an improved graphene-based electrochemical biosensor achieving highly sensitive label-free detection with fM sensitivity.

More specifically the aim of the present invention is to provide an improved biosensor comprising a reduced Graphene Oxide (rGO) screen-printed electrode (SPEs), wherein bio-receptors are covalently bound to the rGO via a functional linker.

The aim of the present invention is also to provide a method for the rGO SPEs surface modification with linker molecules for effective immobilisation of bioreceptors and for improving the performance of biosensors.

According to the present invention, an improved label-free graphene-based electrochemical biosensor is obtained by amine (NH₂) linker modification of the electrodes.

According to an embodiment of the present invention a novel label-free graphene-based electrochemical biosensor is presented, comprising
a substrate covered with reduced Graphene Oxide (rGO) screen-printed electrodes (SPEs),
NH₂ functional groups used as linkers and attached on the surface of printed rGO SPEs via chemisorption,
bio-receptor molecules covalently bonded to the NH₂ linkers,
bovine serum albumin (BSA) configured for saturating of the surface dangling bonds of the rGO SPEs.

According to an embodiment of the present method a novel surface modification technique, hereinafter referred to as the "functionalization" technique, aimed to attach amine (NH₂) linker on the rGO SPE electrode surfaces of the label-free electrochemical biosensor is presented.

According to an embodiment of the present invention, the functionalization technique comprises the step of reacting commercially available rGO SPEs with ammonia solution at room temperature.

According to an embodiment of the present method the NH₂ linkers are preferebly attached on the oxygenated and defect sites of rGO SPE via chemisorption as revealed by XPS and Raman analysis.

According to an example of application of the present method, the novel NH₂ functionlized label-free graphene-based electrochemical biosensor is demonstrated to achieve a limit of detection (LOD) of 9.4 fM for the detection of Aβ₁₋₄₂ biomarkers.

According to another application of the present method a LOD of 14.29 fM is demonstrated to be achieved for DNA methylation biomarker detection.

### BRIEF DESCRIPTION OF THE DRAWINGS

For a better understanding of the present invention, preferred embodiments thereof are now described purely by way of non-limiting example and with reference to the attached drawings, wherein:
- Figure 1 shows schematic illustration of the detection process. (a) Modification of rGO electrode by NH₂ linkers through ammonia chemisorption. (b) Covalent immobilization of antibodies. (c) Blocking via bovine serum albumin (BSA). (d) Binding of antigen.
- Figures 2-3 show concentration calibration curves for Aβ₁₋₄₂ and methylated DNA plotted as normalised peak current vs biomarker concentration on a logarithmic scale.
- Figure 4 is a schematic representation of the fabrication steps comprising the functionalization ones: (a) commercially available rGO SPE; (b) immersion in ammonia solution; (c) incubation in Antibody solution; (d) adding blocking layer (BSA); (e) detection of antigen.

### DETAILED DESCRIPTION OF THE INVENTION

In the recent years variety of diagnostic platforms have been developed to facilitate the monitoring of chronic diseases. However, none of them have demonstrated to be rapid, sensitive, minimally invasive, and reliable enough to be clinically applicable.

Graphene or reduced graphene oxide (rGO) based label-free biosensor have demonstrated to have the ability to detect biomarkers in a cost and time efficient way. The graphene-based biosensor performance in term of sensitivity is far most important to increase the probability of attaching higher number of bio-receptors to capture the target biomarker.

To achieve better performance, the immobilisation of bio-receptors on the sensor surfaces is often achieved via specific linker chemistry. Linker conjugation with nanomaterials such as graphene and reduced graphene oxide (rGO) has been shown to improve the performance of biosensors.

In the client's prior art, the use of Pyrenebutyric acid N-hydroxysuccinimide ester (Pyr-NHS) linker for improving the performance of biosensors has been explored. In this study it has been shown that the modification of graphene/rGO dual-layer with Pyr-NHS is an effective and reliable tool for the detection of Aβ₁₋₄₂ biomarkers from plasma samples. The biosensor has also shown sensitivity and reliability for the detection of Clusterin, with detection limit of the order of pM.

The present invention is aimed to provide a new approach, consisting of directly attaching primary amines (NH₂) linkers on sensor surface. The NH₂ linker has small dimensions and therefore is more densely attached to the surface providing more numerous binding sites.

As will be discussed in detail in the following, an embodiment of the present solution envisages a label-free electrochemical biosensor comprising reduced Graphene Oxide (rGO) screen-printed electrodes (SPEs) surface modified by attaching of NH₂ linkers.

In particular, as will be detailed in the following, one aspect of the present solution envisages use of a novel functionalization technique to attach amine (NH₂) linker on the graphene-based sensor surfaces.

Advantage of the present invention is an increased performance of the biosensor by implementing a small NH₂ linker. In this way it is possible to achieve highly sensitive label-free detection with fM sensitivity.

As used herein, the term "bioreceptors" also reported as "bio-receptors" or "capture molecules" generally refers to a substance that can covalently bind another substance. This bond is preferably selective. Examples of capture molecules are antibodies. The term " biomarker" includes any compound or substance that can be an antigen of an antibody or a substrate of another capture molecule.

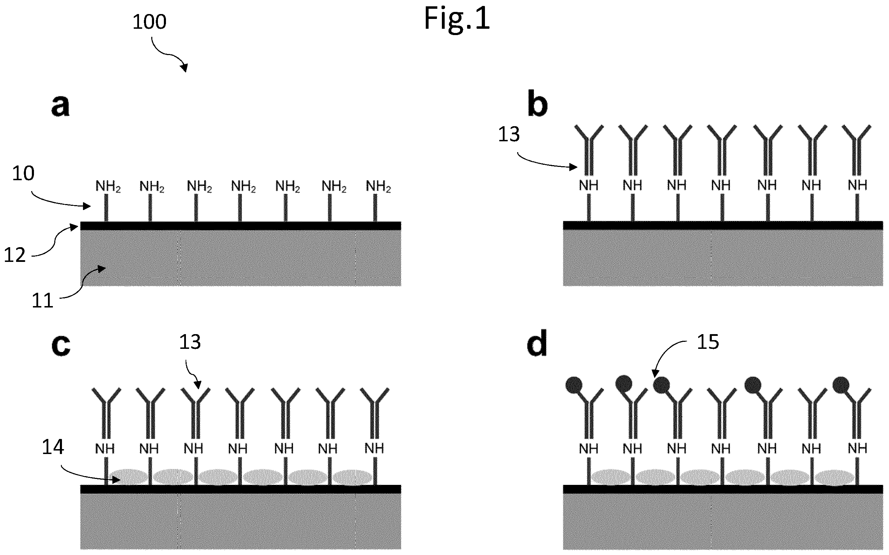

In Figure 1 is depicted a schematic illustration of the detection process for antigens according to the present invention. As shown in Fig.1a, the label-free electrochemical biosensor (100) is functionalized by attaching the NH₂ linkers (10) to the rGO screen-printed electrodes (12) surface. The NH₂ linker is immobilised on the rGo surface via chemisorption of ammonia (NH₃) as revealed by XPS examinations. In Fig.1b the bioreceptors, that is the antibodes (13), are covalently immobilized by the NH₂ linkers. In Fig.1c BSA, (14), is used for saturating the free surface bonds. In Fig. 1d the biomarker to be detected, that is the antigen (15), is bound to its immobilized antibody.

### Biosensor fabrication.

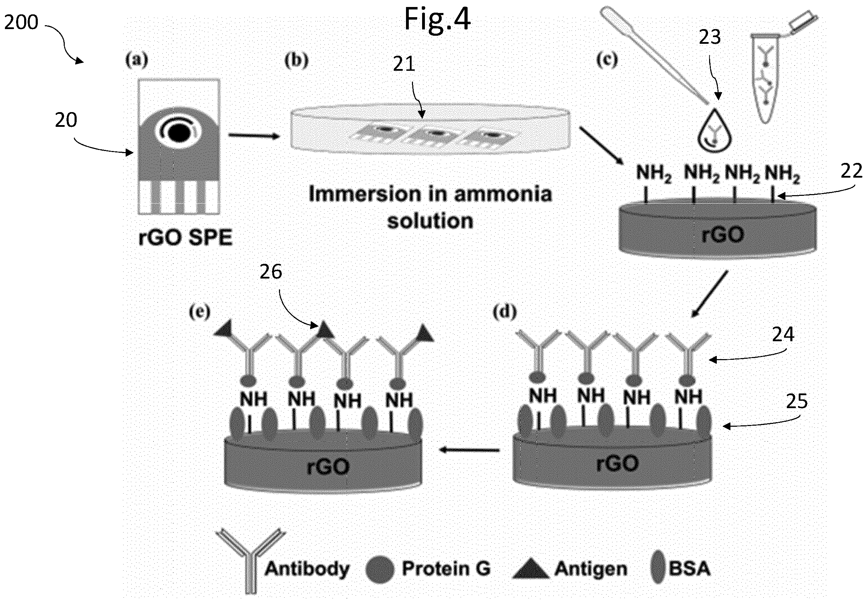

In Fig.4 a fabrication process (200) of the biosensors according to a preferred embodiment of the present invention is showed. The process used to fabricate the biosensor can be summarized as follows:
1. Firstly, commercially available screen-printed electrode (SPE) coated with the reduced Graphene Oxide (rGO), (20), is immersed in ammonia solution (21) (containing 10-50% of NH₃) for a time ranging from 30 min up to 6 hours at room temperature. This step is referred to as ammonia treatment, Fig.4(a-b). During this step, the NH₂ linkers (22) are immobilised on the rGo surface via chemisorption of ammonia (NH₃).
2. Subsequently, a defined antibody solution (23) of antibody and protein G is prepared by vortex mixer and allowed to sit at room temperature for a time interval ranging from 30 min up to 3 hours to encourage strong bond formation,(Fig.4c).
3. Then, the antibody molecules (24) are covalently bound to the NH₂ linkers (22) on the rGO surface by incubating the ammonia treated rGO SPEs in the antibody solution mixture for a time interval ranging from 30 min to 6 hours at room temperature.
4. After that, a blocking layer is deposited to saturate the surface free bonds and reducing the possibility of non-specific binding. For example, a 1% of Bovine Serum Albumina (BSA), (25), in Phosphate-buffered saline (PBS) is drop casted onto the SPEs for 15 minutes followed by rinsing with PBS, (Fig.4d) .
5. Finally, the antigen molecules (26) are attached to the antibodies (Fig.4e) by drop casting the desired solution (for example a diluited human plasma in PBS) on the biosensor and incubating at room temperature for a time interval ranging from 30 min to 4 hours.

### Electrochemical Measurement

The biomarkers detecting process of the biosensor is provided through the variation of its electrical properties. For example, a variation in the concentration of the biomarker is detected by a measurable variation of the electrical current, or electrical voltage, or electrical capacitance, or electrical inductance, or electrical resistance, or the electrical impedance of the biosensor. Differential pulse voltammetry (DPV) and cyclic voltammetry (CV) measurement techniques are usually performed at room temperature for electrochemical measurements. For example, DPV is a technique that involves applying amplitude potential pulses on a linear ramp potential. A base potential applied to the electrode of the potentiostat is increased between pulses with equal increments. The current is immediately measured before the pulse application and at the end of the pulse, and the difference between them is recorded.

### Example.

The label-free detection of biomarkers employing the new NH₂ linker is demonstrated using screen-printed rGO electrodes for the detection of Aβ₁₋₄₂ proteomic biomarkers of Alzheimer's disease (AD) and DNA methylation.

The Aβ₁₋₄₂ antibody solution (20 µg/mL) was mixed in the ratio of 70:30 with protein G (20 µg/mL). The desired dilutions of Aβ₁₋₄₂ peptides were freshly prepared in PBS by vortex mixing for 20 seconds. The prepared peptides were kept on ice during the experiments. 10 µL of each peptide solution was drop casted on the biosensor and incubated at room temperature for 1 hour. After that, the sensor was rinsed with PBS to remove any unbound peptides.

Differential pulse voltammetry (DPV) measurement is used to characterize the performance of the biosensors and is performed at room temperature. For this specific case DPV was recorded from -0.15 V to +0.45 V at a scan rate of 50 mV.s⁻¹, pulse amplitude of 50 mV, step potential of 10 mV and pulse period of 0.4 s. The data was acquired at a working potential of -165 mV.

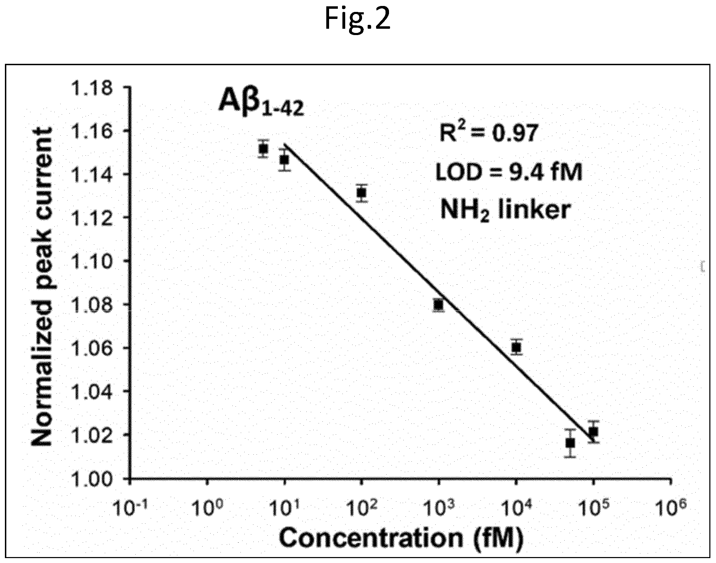

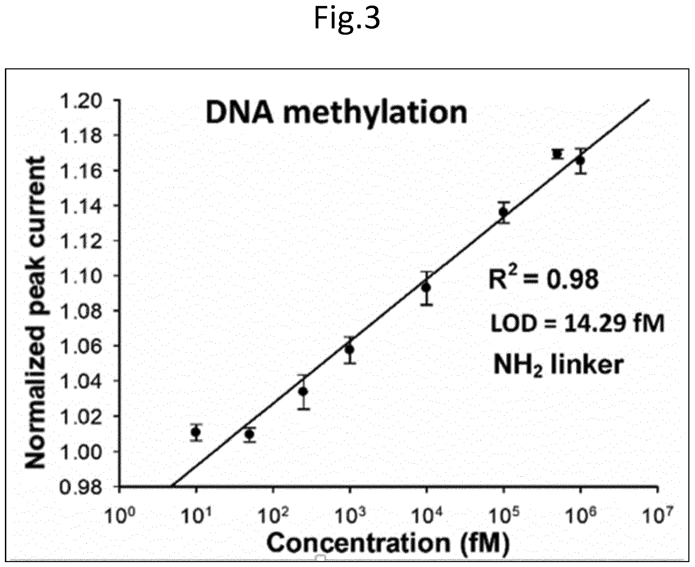

Typical peak-current versus biomarker concentration plots are shown in Fig.2 for the detection of Aβ₁₋₄₂ and in Fig.3 for DNA methylation. As shown in Fig.2, DPV measurement shows a greater linear response range of biomarker concentrations with excellent regression coefficients (R²) of around 0.97. The estimated limit of detection (LOD) is 9.4 fM for detection of Aβ₁₋₄₂ biomarker and 14.29 fM for DNA methylation, which is two orders of magnitude better than the prior art. We attribute these improvements to the higher density of antibodies immobilized onto the rGO electrodes, which is achieved via the chemisorption of ammonia. These molecules effectively attach more target-receptor molecules on the electrode surface, leading to remarkable performance in limit of detection and linear dynamic sensing range of rGO electrodes.

### Reaction mechanism

The surface reactions of rGO with NH₂ linker during the functionalization step has been analysed using XPS and Raman techniques. In particular, as detailed below, the functionalization of rGO surface electrode of the biosensor takes place by chemisorption mechanism of NH₂ groups. According to an embodiment of the present method, during the ammonia treatment step, the ammonia solution (NH₃.H₂O) reacts with rGO by the removal (abstraction) of hydrogen (H) atom and the formation of new chemical bonds between the rGO surface with NH₂ groups (chemisorption of NH₂ groups). According to this theory, within the rGO the H atoms in NH₃/NH₄⁺ are attracted from the highly electronegative oxygen atoms bound to the Carbon in epoxide (C-O), in carboxyl (O-C=O) and in hydroxyl (O-H) groups. As a conseguense the H atom combines with C-O groups forming C-OH and with O-H groups forming H₂O and dissociates from NH₃/NH₄⁺ groups forming NH₂. The NH₂ groups react with C atoms on oxygenated functional groups and defect sites giving rise to the functionalizing of rGO. Due to the presence of defect sites a state of non-equilibrium is generated, so they readily bind with NH₂ to rebalance it.

The honeycomb structure of rGO is not affected by all these surface reactions. Also, because one SPE is made of a large number of rGO flakes, there is a lot of NH₂ groups attached on the SPE. As a conseguence, a lot of binding sites for antibody are formed, improving the limit of detection of the biosensor.

Finally, it is clear that numerous modifications and variants can be made to the present invention, all falling within the scope of the invention, as defined in the appended claims.

## Claims

1. A label-free electrochemical biosensor (100) comprising:
a. reduced Graphene Oxide (rGO) screen-printed electrodes (SPEs) (12) disposed on a biosensor substrate (11),
b. primary amines (NH₂) linkers (10) with NH2-groups attached to said SPEs (12),
c. bioreceptor molecules (13) covalently bound to said (NH₂) linkers (10),
d. blocking layer (14) deposited on said rGO SPEs (12)
wherein said rGO SPEs (12) and said NH2-groups, are covalently bonded through chemisorption mechanism by immersion of said rGO SPEs (12) in ammonia solution at room temperature.

2. Label-free electrochemical biosensor (100) according to claim 1 wherein said bioreceptor molecules (13) are antibody molecules.

3. Label-free electrochemical biosensor (100) according to claim 1 wherein said blocking layer (14) is Bovine Serum Albumin (BSA).

4. A method of fabrication of a label-free electrochemical biosensor (100-200), said method comprising the following process steps:
a. covering a biosensor substrate with Graphene-based reduced Oxide (rGO) screen-printed electrodes (SPEs) (20),
b. functionalizing said rGO SPEs (20) by attaching primary amines (NH₂) linkers (22) thereon,
c. covalently bonding bioreceptor molecules (24) to said (NH₂) linkers (22),
d. depositing a blocking layer (25) on said rGO SPEs (20)
wherein functionalizing said rGO SPEs (20) comprises the step of immersing said Graphene based rGO SPEs (20) for a time range 0,5-6 hours in ammonia solution (21) containing 10-50% of NH3 at room temperature.

5. The method according to claim 4 wherein said primary amines NH₂ linkers attach on to said rGO SPEs (20) surface by chemisorption mechanism.

6. The method according to claim 5 wherein said chemisorption mechanism comprises the reaction of said ammonia solution (NH₃.H₂O) (21) with said rGO SPEs (20) with the removal (abstraction) of hydrogen (H) atom and the formation of new chemical bonds between said rGO SPEs (20) surface and NH₂ linkers.

7. The method according to claim 4 wherein the step of covalently bonding bioreceptor molecules (24) to said (NH₂) linkers (22) comprises:
a. Preparing an antibody solution (23) by vortex mixer and letting it sit at room temperature for a time range 0,5-3 hours,
b. incubating the functionalized Graphene-based rGO SPEs (20) in said antibody solution (23) mixture for a time range 0,5-6 hours at room temperature.

8. The method according to claim 4 wherein the step of depositing a blocking layer (25) on said rGO SPEs (20) comprises the step of:
a. drop casting a 1% of Bovine Serum Albumina (BSA), (25), in Phosphate-buffered saline (PBS) onto the functionalized rGO SPEs (20) for 15 minutes;
b. rinsing with Phosphate-buffered saline PBS.

9. A method to use the label-free electrochemical biosensor (100-200) according to claim 1, said method comprising
a. drop casting a body-fluid based biomarker solution on said biosensor (100-200) and incubating at room temperature for a time range 0,5-4 hours,
b. detecting the variation of the electrical properties of said biosensor (100-200) induced by the contact with said body-fluid based biomarker solution.

10. The method according to claim 9 wherein said body-fluid based biomarker solution is a diluited human plasma in PBS.

## Patentansprüche

1. Etikettenfreier elektrochemischer Biosensor (100), umfassend:
a. Siebgedruckte Elektroden (SPE) (12) mit reduziertem Graphenoxid (rGO), auf einem Substrat (11) eines Biosensors angeordnet,
b. Linker (10) aus primärem Amin (NH₂) mit an die SPE (12) gebundenen NH2-Gruppen,
c. Biorezeptormoleküle (13), die kovalent mit dem Linker (NH₂) (10) gebunden sind,
d. eine auf dem rGO SPE (12) abgeschiedene Blockierschicht (14)
wobei die rGO SPE (12) und die NH2-Gruppen durch chemische Absorptionsmechanismen beim Eintauchen von rGO SPE (12) in Ammoniaklösung bei Raumtemperatur, kovalent gebunden sind.

2. Etikettenfreier elektrochemischer Biosensor (100) nach Anspruch 1, wobei die Moleküle des Biorezeptors (13) Antikörpermoleküle sind.

3. Etikettenfreier elektrochemischer Biosensor (100) nach Anspruch 1, wobei die Blockierungsschicht (14) bovines Rinderserumalbumin (BSA) ist.

4. Verfahren zur Herstellung eines etikettenfreien elektrochemischen Biosensors (100-200), wobei das Verfahren die folgenden Verfahrensschritte umfasst:
a. Bedecken eines Biosensorsubstrats mit siebgedruckten Elektroden (SPE) (20) mit reduziertem Graphenoxid (rGO),
b. Funktionalisieren der rGO-SPEs (20), indem primäre Amin-Linker (NH₂) (22) daran befestigt werden,
c. kovalentes Verbinden von Molekülen eines Biorezeptors (24) mit dem Linker (NH2) (22),
d. Abscheiden einer Sperrschicht (25) auf dem rGO SPE (20),
wobei die Funktionalisierung dieser rGO SPE (20) den Schritt des Eintauchens dieser auf Graphen basierenden rGO SPE (20) für einen Zeitraum von 0,5 bis 6 Stunden in eine Ammoniaklösung (21) mit 10-50 % NH3 bei Raumtemperatur umfasst.

5. Verfahren nach Anspruch 4, wobei sich der NH₂-Linker von primären Aminen durch einen chemischen Absorptionsmechanismus an die Oberfläche des rGO SPE (20) anlagert.

6. Verfahren nach Anspruch 5, wobei der chemische Absorptionsmechanismus die Reaktion der Ammoniaklösung (NH₃.H₂O) (21) mit dem rGO SPE (20) unter Entfernung (Abstraktion) eines Atoms von Wasserstoff (H) und die Bildung neuer chemischer Bindungen zwischen der Oberfläche des rGO SPE (20) und den NH₂-Linker umfasst.

7. Verfahren nach Anspruch 4, wobei der Schritt der kovalenten Verbindung der Moleküle des Biorezeptors (24) mit diesem Linker (NH₂) (22) umfasst:
a. Bereiten mit einem Zyklonmischer einer Antikörperlösung (23) vor und diese für einen Zeitraum von 0,5 bis 3 Stunden bei Raumtemperatur absetzen lassen.
b. Inkubieren der funktionalisierten graphenbasierten rGO SPEs (20) in der Mischung aus Antikörperlösungen (23) für einen Zeitraum von 0,5 bis 6 Stunden bei Raumtemperatur.

8. Verfahren nach Anspruch 4, wobei dem der Schritt der Abscheidung einer Sperrschicht (25) auf der rGO SPE (20) den Schritt umfasst:
a. Gießen von 1 % Rinderserumalbumin (BSA) (25) 15 Minuten lang tropfenweise in eine phosphatgepufferte Salzlösung (PBS) auf das funktionalisierte rGO SPE (20),
b. Spülen mit phosphatgepufferter PBS-Salzlösung.

9. Verfahren zur Verwendung eines etikettsfreien elektrochemischen Biosensors (100-200) nach Anspruch 1, wobei das Verfahren Folgendes umfasst:
a. tropfenweises Gießen von einer Biomarkerlösung auf Basis einer Körperflüssigkeit auf diesen Biosensor (100-200) und bei Raumtemperatur für einen Zeitraum von 0,5 bis 4 Stunden inkubieren,
b. Bewerten der Variation der elektrischen Eigenschaften des Biosensors (100-200) in Kontakt mit der Biomarkerlösung basierend auf einer Körperflüssigkeit.

10. Verfahren nach Anspruch 9, wobei die Lösung des Biomarkers auf Basis einer Körperflüssigkeit ein in PBS verdünntes menschliches Plasma ist.

## Revendications

1. Biocapteur électrochimique sans étiquette (100) comprenant :
a. des électrodes sérigraphiés (SPE) (12) avec un réduit oxyde de graphène (rGO) disposés sur un substrat (11) d'un biocapteur,
b. lieur (10) d'amine primaire (NH2) avec les groupes NH2 fixés auxdits SPE (12),
c. molécules de bio-récepteurs (13) liées de manière covalente auxdits lieurs (NH2) (10),
d. une couche de blocage (14) déposée sur lesdits rGO SPE (12)
dans lequel les rGO SPE (12) et les groupes NH2 sont liés de manière covalente pour les mécanismes d'absorption chimique au cours de l'immersion des rGO SPE (12) dans une solution d'ammoniac à température ambiante.

2. Biocapteur électrochimique sans étiquette (100) selon la revendication 1, dans lequel lesdites molécules du bio-récepteur (13) sont des molécules d'anticorps.

3. Biocapteur électrochimique sans étiquette (100) selon la revendication 1, dans lequel la couche de blocage (14) est de l'albumine de sier bovin (BSA).

4. Méthode pour fabriquer un biocapteur électrochimique sans étiquette (100-200), la méthode comprenant les étapes suivantes du processus :
a. couvrir un substrat de biocapteur avec des électrodes sérigraphiés (SPE) (20) avec un réduit oxyde de graphène (rGO),
b. fonctionnaliser les rGO SPE (20) en fixant des lieurs (22) d'amine primaire (NH₂) sur les mêmes,
c. unir de manière covalente des molécules d'un bio-récepteur (24) auxdits lieurs (NH₂) (22),
d. déposer une couche de blocage (25) sur lesdits rGO SPE (20)
dans lequel la fonctionnalisation de ces rGO SPE (20) comprend la phase d'immersion de ces rGO SPE (20) à base de graphène pour une période de temps de 0,5 à 6 heures dans une solution d'ammoniac (21) contenant 10-50% de NH3 à température ambiante.

5. Méthode selon la revendication 4, dans lequel lesdits lieurs NH₂ d'amines primaires se fixent sur la surface desdits rGO SPE (20) par un mécanisme d'absorption chimique.

6. Méthode selon la revendication 5, dans laquelle le mécanisme d'absorption chimique comprend la réaction de la solution d'ammoniac (NH₃.H₂O) (21) avec les rGO SPE (20) avec la suppression (abstraction) d'un atome d'hydrogène (H) et la formation de nouveaux liens chimiques entre ladite surface des rGO SPE (20) et le lieur NH₂.

7. Méthode selon la revendication 4, dans laquelle l'étape de lier de manière covalente les molécules du bio-récepteur (24) avec ce lieur (NH₂) (22) comprend:
a. préparer une solution d'anticorps (23) à l'aide d'un mélangeur cyclonique et la laisser déposer à température ambiante pendant une période de temps de 0,5 à 3 heures,
b. incuber les rGO SPE (20) à base de graphène fonctionnalisés dans ledit mélange de solutions d'anticorps (23) pendant une période de temps de 0,5 à 6 heures à température ambiante.

8. Méthode selon la revendication 4, dans laquelle la phase de dépôt d'une couche de blocage (25) sur les rGO SPE (20) comprend l'étape de:
a. verser goutte à goutte 1% de l'albumine de sier bovin (BSA) (25) dans une solution saline tamponnée avec du phosphate (PBS) sur les rGO SPE (20) fonctionnalisés pendant 15 minutes,
b. rincer avec une solution saline de PBS tamponnée avec du phosphate.

9. Méthode d'utilisation d'un biocapteur électrochimique sans étiquette (100-200) selon la revendication 1, la méthode comprenant
a. verser goutte à goutte une solution de bio-marqueur à base d'un fluide corporel sur ce biocapteur (100-200) et incuber à température ambiante pendant une période de 0,5 à 4 heures,
b. évaluer la variation des propriétés électriques du biocapteur (100-200) en contact avec la solution du bio-marqueur basée sur un fluide corporel.

10. Méthode selon la revendication 9 dans laquelle la solution du bio-marqueur à base d'un fluide corporel est un plasma humain dilué dans du PBS.
